# EUROPEAN PATENT SPECIFICATION

(11) **EP 4 324 419 B1**
(45) Date of publication and mention of the grant of the patent: **10.09.2025**
(21) Application number: 23800297.6
(22) Date of filing: 20.06.2023
(51) Int. Cl.: A61B 18/20

(54) **HAIR REMOVAL DEVICE**
HAARENTFERNUNGSINSTRUMENT
DISPOSITIF D'ÉPILATION

(30) Priority: 20.06.2022 CN 202221551332 U; 20.06.2022 CN 202221550413 U; 20.06.2022 CN 202221551331 U
(43) Date of publication of application: 21.02.2024
(73) Proprietor: Shenzhen Ulike Smart Electronics Co., Ltd., Shenzhen, Guangdong 518000 (CN)
(72) Inventor: FANG, Shaoqing, Shenzhen, Guangdong 518000 (CN)
(74) Representative: Valet Patent Services Limited
(86) International application number: PCT/CN2023/101524
(87) International publication number: WO 2023/246817

(56) References cited:
- WO-A1-2022/082837
- WO-A1-2022/116470
- CN-A- 111 821 019
- CN-A- 111 973 266
- CN-A- 112 168 344
- CN-A- 113 729 930
- CN-A- 114 176 765
- CN-U- 203 861 346
- CN-U- 213 156 396
- CN-U- 214 908 023
- CN-U- 217 907 973
- CN-U- 218 045 321
- CN-U- 218 773 935

## Description

### TECHNICAL FIELD

This application relates to the field of hair removal instrument technologies, and specifically, to a hair removal instrument.

### BACKGROUND

Photon hair removal is a new hair removal technology and a new cosmetic method on the market. Photon hair removal refers to destroying hair follicles by using a special wavelength of intense pulsed light to achieve an effect of permanent hair removal with advantages such as a fast speed, a good effect, high safety, no side effects, and painlessness. Therefore, photon hair removal is widely welcomed in the beauty and medical industries. Hair removal devices are disclosed in CN213156396 and CN114176765, for instance.

In an existing hair removal instrument, a turbo fan is basically used to perform air-cooled heat dissipation on heat generated inside the hair removal instrument. However, a safety hazard is easily caused when a temperature inside the hair removal instrument is excessively high.

In conclusion, how to prevent a temperature inside a hair removal instrument from being excessively high and reduce safety hazards already becomes a technical problem to be resolved urgently.

### SUMMARY

The invention and its most advantageous embodiments are defined in the appended set of claims. An embodiment of this application provides a hair removal instrument. The hair removal instrument includes: a cooling driver, provided with a driving port, where the cooling driver is capable of outputting a cooling medium through the driving port; a housing, where the cooling driver is fastened in the housing, and the housing is provided with an air outlet, a cooling channel butt-jointed to the driving port and in communication with the air outlet, and a light outlet in communication with the cooling channel; a hair removal apparatus, located in the housing and fastened in the cooling channel; a cold compress part, fastened on the light outlet and opposite to the hair removal apparatus, to receive light emitted by the hair removal apparatus and emit the light; a heat conduction part, fastened in the housing, where one end of the heat conduction part is located in the cooling channel, and the other end of the heat conduction part is connected to the cold compress part; a temperature control apparatus, mounted in the housing and located on one side that is of the heat conduction part and that is away from the cooling driver, and close to the air outlet; a control board, fastened in the housing, wherein all of the cooling driver, the hair removal apparatus, the cold compress part, and the temperature control apparatus are electrically connected to the control board.

In the hair removal instrument provided in this application, when the temperature control apparatus detects that a temperature at the air outlet is excessively high, the temperature control apparatus transmits information to the control board, and the control board powers off the hair removal apparatus, so that the hair removal apparatus stops operating, thereby avoiding a safety hazard caused by an excessively high temperature inside the housing.

### BRIEF DESCRIPTION OF DRAWINGS

To more clearly describe the technical solutions in the embodiments of this application, the accompanying drawings required for the descriptions of the embodiments are briefly described below. Apparently, the accompanying drawings described below are merely some embodiments of this application. A person of ordinary skill in the art may further derive other accompanying drawings based on these accompanying drawings without creative efforts.
FIG. 1 is a schematic diagram of a structure of an embodiment of a hair removal instrument according to this application;
FIG. 2 is a schematic diagram of a structure of an embodiment obtained after a power adapter and a wire are removed from a hair removal instrument according to this application;
FIG. 3 is a schematic diagram of an exploded structure of an embodiment obtained after a power adapter and a wire are removed from a hair removal instrument according to this application;
FIG. 4 is a schematic diagram of an exploded structure of another embodiment obtained after a power adapter and a wire are removed from a hair removal instrument according to this application;
FIG. 5 is a schematic diagram of an exploded structure of still another embodiment obtained after a power adapter and a wire are removed from a hair removal instrument according to this application;
FIG. 6 is a schematic diagram of an exploded structure of yet another embodiment obtained after a power adapter and a wire are removed from a hair removal instrument according to this application;
FIG. 7 is a schematic diagram of an exploded structure of yet another embodiment obtained after a power adapter and a wire are removed from a hair removal instrument according to this application;
FIG. 8 is a schematic diagram of a structure of an embodiment obtained after a bottom housing, an air guide housing, a capacitor, and a cooling driver are combined according to this application;
FIG. 9 is a schematic diagram of a structure of an embodiment obtained after a control board, an air guide housing, a heat conduction part, and a cooling driver are combined according to this application;
FIG. 10 is a schematic diagram of a structure of an embodiment obtained after a control board, a hair removal apparatus, and a cooling driver are combined according to this application;
FIG. 11 is a schematic diagram of a structure of an embodiment of a separator according to this application;
FIG. 12 is a schematic diagram of a structure of an embodiment of a cooling driver according to this application;
FIG. 13 is a schematic diagram of a structure of another embodiment obtained after a power adapter and a wire are removed from a hair removal instrument according to this application; and
FIG. 14 is a schematic diagram of an exploded structure of FIG. 13 according to this application.

### DESCRIPTION OF EMBODIMENTS

The following further describes this application in detail with reference to the accompanying drawings and the embodiments. It should be particularly noted that the following embodiments are merely used for describing this application but not limiting the scope of this application. Similarly, the following embodiments are merely some rather than all of the embodiments of this application. All other embodiments derived by a person of ordinary skill in the art without creative efforts shall fall within the protection scope of this application.

Referring to the "embodiments" in this application means that a particular feature, structure, or characteristic described with reference to the embodiments may be included in at least one embodiment of this application. A person skilled in the art explicitly and implicitly understands that the embodiments described in this application may be combined with other embodiments.

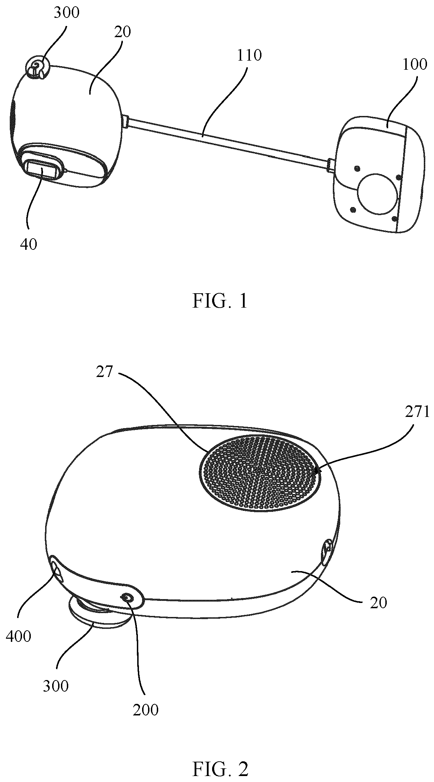

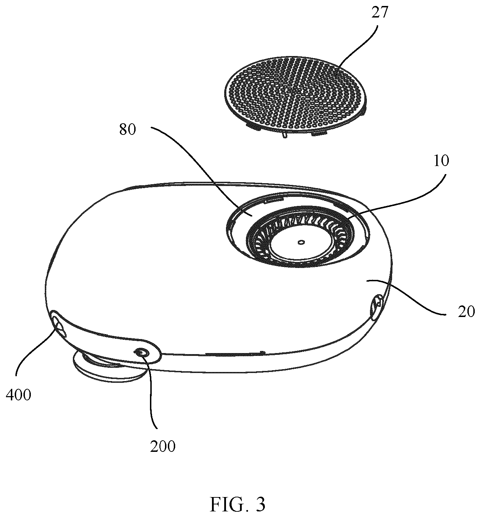

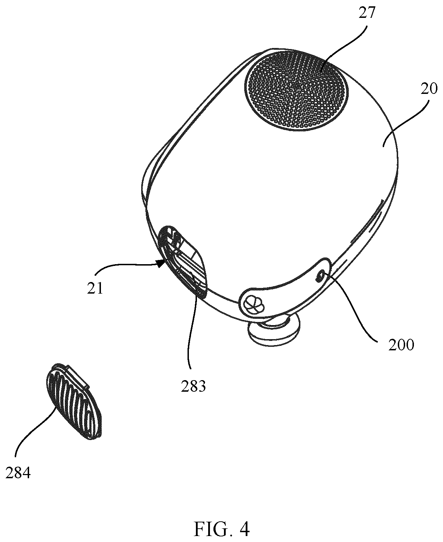

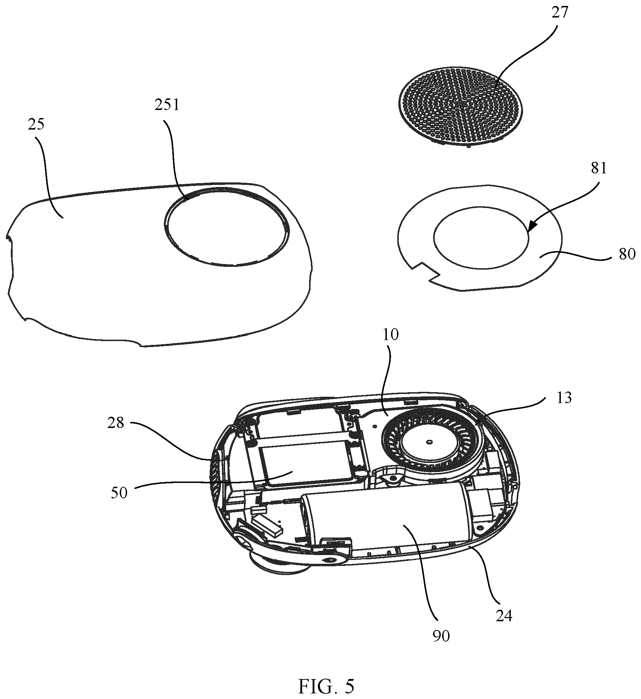

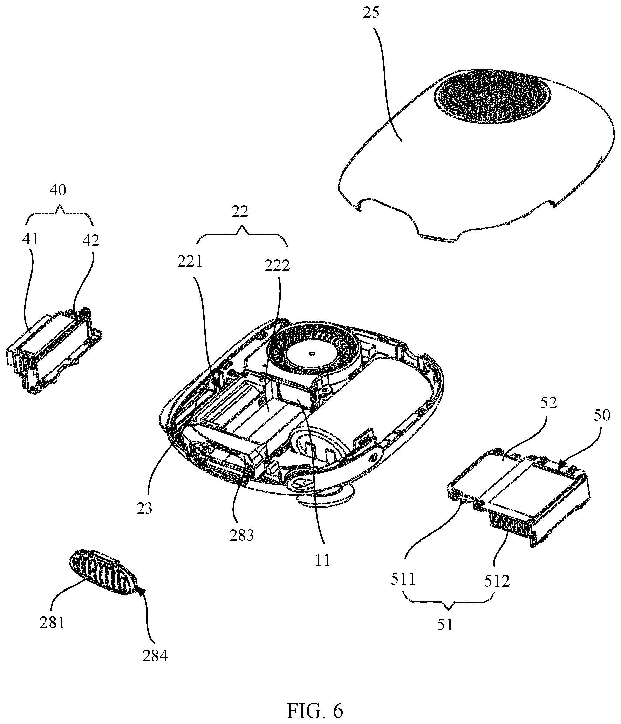

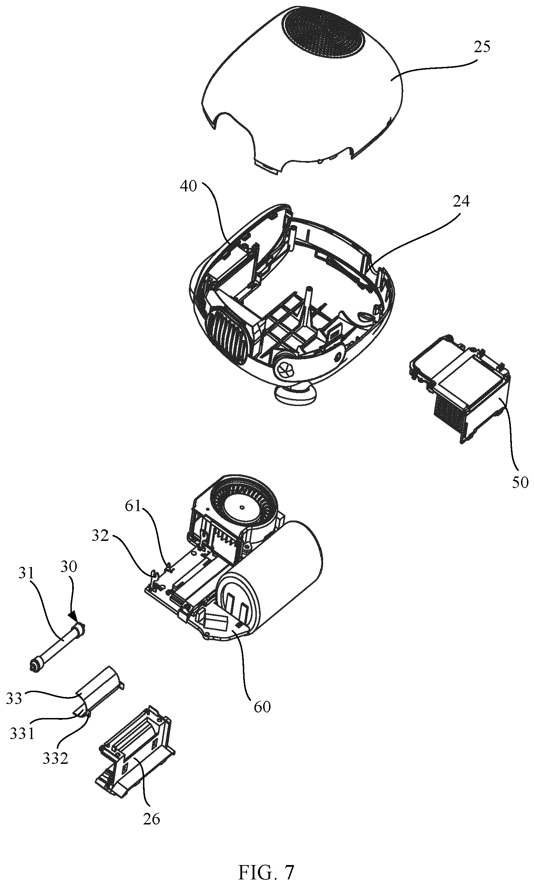

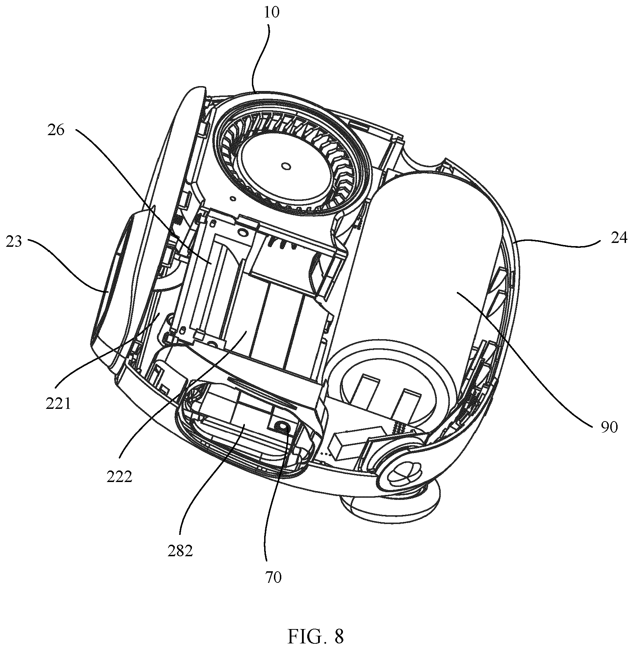

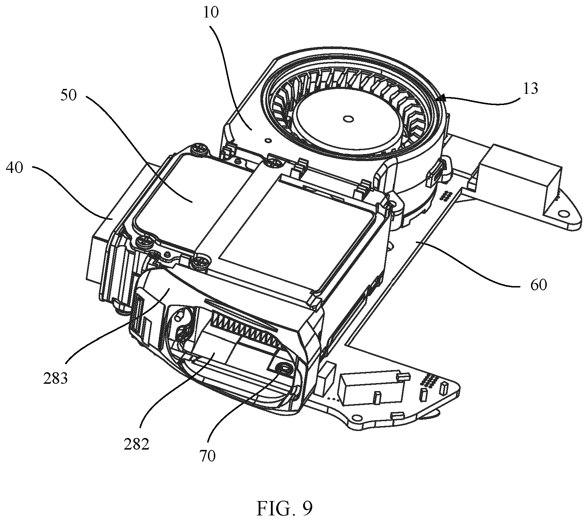

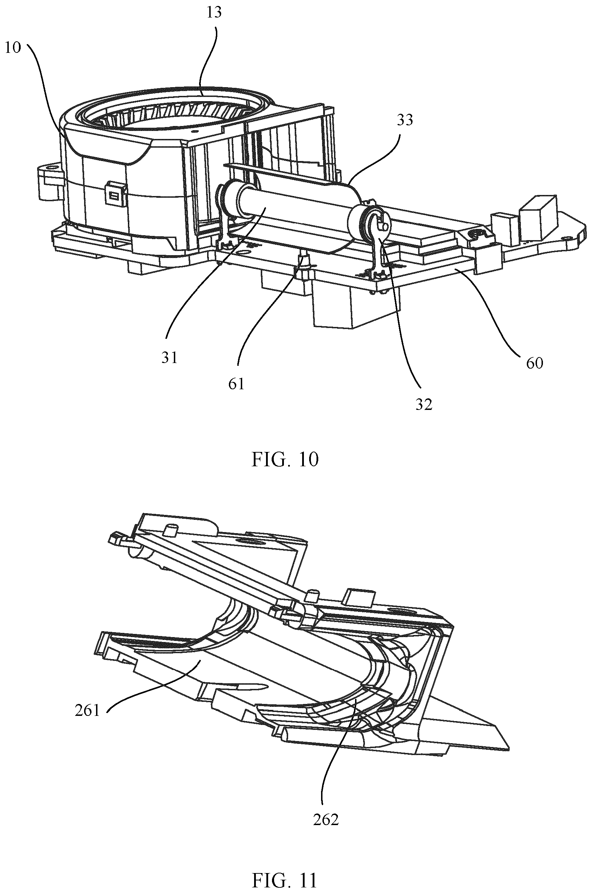

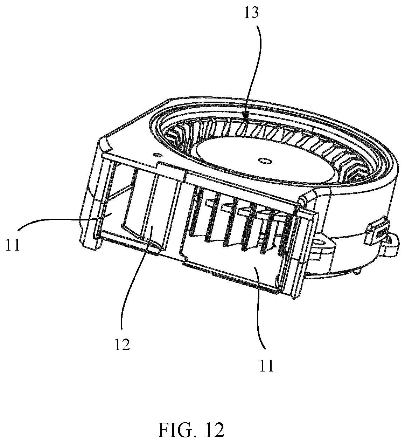

Refer to FIG. 1 to FIG. 12. An embodiment of this application discloses a hair removal instrument that can prevent an internal temperature from being excessively high. The hair removal instrument that can prevent an internal temperature from being excessively high is hereinafter referred to as a hair removal instrument for short. The hair removal instrument includes a cooling driver 10, a housing 20, a hair removal apparatus 30, a cold compress part 40, a heat conduction part 50, a control board 60, and a temperature control apparatus 70.

The cooling driver 10 is provided with a driving port 11, the cooling driver 10 may output a cooling medium through the driving port 11, and the cooling driver 10 is fastened in the housing 20. The housing 20 is provided with an air outlet 21, a cooling channel 22 butt-jointed to the driving port 11 and in communication with the air outlet 21, and a light outlet 23 in communication with the cooling channel 22. The cooling channel 22 is located at a middle part of the housing 20, the air outlet 21 and the light outlet 23 are located at edge positions of the housing 20, and the air outlet 21 and the driving port 11 are disposed opposite to each other.

The hair removal apparatus 30 is located in the housing 20 and is fastened on an inner wall of the cooling channel 22. The cold compress part 40 is fastened on the light outlet 23 and is opposite to the hair removal apparatus 30, so that the cold compress part 40 receives light, namely, laser light, emitted by the hair removal apparatus 30 and emits the light. The hair removal apparatus 30 irradiates hair follicles with the light through skin to remove hair. The cold compress part 40 can quickly cool to relieve burning feeling caused by the light, to ensure comfort.

The cold compress part 40 may be specifically sapphire, K9 glass, crystal glass, or any light-transmission crystalline material, and may preferably be a sapphire material.

The heat conduction part 50 is fastened in the housing 20, one end of the heat conduction part 50 is located in the cooling channel 22, and the other end of the heat conduction part 50 is connected to the cold compress part 40. The heat conduction part 50 may transmit heat on the cold compress part 40 to the heat conduction part 50. The cooling medium may be used to dissipate the heat on the heat conduction part 50 from the air outlet 21, so that heat in the entire housing 20 is reduced. The control board 60 is fastened in the housing 20.

The temperature control apparatus 70 is preferably a temperature sensor, a temperature controller, or the like. The temperature control apparatus 70 is mounted in the housing 20 and is located on one side that is of the heat conduction part 50 and that is away from the cooling driver 10. All of the cooling driver 10, the hair removal apparatus 30, the cold compress part 40, and the temperature control apparatus 70 are electrically connected to the control board 60. The temperature control apparatus 70 may preferably be close to the air outlet 21. When the temperature control apparatus 70 detects that a temperature at the air outlet 21 is excessively high, the temperature control apparatus 70 transmits information to the control board 60, and the control board 60 powers off the hair removal apparatus 30, so that the hair removal apparatus 30 stops operating, thereby avoiding a safety hazard caused by an excessively high temperature inside the housing 20. For example, the temperature at the air outlet 21 is excessively high when the cold compress part 40 or the cooling driver 10 is damaged. In this case, the burning feeling caused on the skin of a human body by the laser light cannot be quickly transferred. Consequently, the skin of the human body is hurt.

In this embodiment, the hair removal apparatus 30 includes a filament-free lamp tube 31, a lamp holder 32, and a reflector cup 33 made of an electrically conductive material. Both the lamp holder 32 and the reflector cup 33 are disposed in the cooling channel 22. The lamp holder 32 is fastened on the control board 60. The reflector cup 33 is detachably mounted on the housing 20. The filament-free lamp tube 31 is mounted on the lamp holder 32 and abuts against the reflector cup 33. The reflector cup 33 is located on one side that is of the filament-free lamp tube 31 and that is away from the cold compress part 40. The reflector cup 33 reflects and focuses light of the filament-free lamp tube 31 on the cold compress part 40, and the reflector cup 33 is electrically connected to the control board 60. The lamp holder 32 provides the filament-free lamp tube 31 with a direct-current voltage. However, the filament-free lamp tube 31 does not light up in this case. The reflector cup 33 serves as a trigger electrode, and the filament-free lamp tube 31 can be triggered and controlled to light up when the control board 60 provides the filament-free lamp tube 31 with a high-voltage pulse by using the reflector cup 33.

In some embodiments, the control board 60 is welded with an elastic conductive member 61. The reflector cup 33 is fastened on an inner wall of the housing 20. The reflector cup 33 is pressed against the elastic conductive member 61. The reflector cup 33 is electrically connected to the control board 60 by using the elastic conductive member 61. The elastic conductive member 61 may be a spring sheet or an assembly including a spring. The elastic conductive member 61 has a contact pressure with the reflector cup 33 on a premise of being electrically connected to the control board 60. This ensures connection effectiveness, a solid structure, and high stability. In addition, the elastic conductive member 61 improves assembly and maintenance efficiency of the hair removal instrument.

The cooling channel 22 includes a first cooling duct 221 and a second cooling duct 222. The cooling channel 22 is a double-duct channel. The first cooling duct 221 and the second cooling duct 222 are disposed in parallel, one end of the first cooling duct 221 and one end of the second cooling duct 222 are butt-jointed to the driving port 11, and the other end of the first cooling duct 221 and the other end of the second cooling duct 222 are butt-jointed to the air outlet 21. The hair removal apparatus 30 is located in the first cooling duct 221. A part of the heat conduction part 50 is located in the second cooling duct 222. Heat dissipation of the heat conduction part 50 and heat dissipation of the hair removal apparatus 30 are separately performed, thereby effectively improving overall cooling efficiency inside the hair removal instrument. A baffle plate 12 configured to limit a capacity of a cooling medium entering the first cooling duct 221 is obliquely disposed on an inner wall of the driving port 11. The driving port 11 is butt-jointed to the first cooling channel 221 and the second cooling channel 222, so that the cooling driver 10 drives an external cooling medium to separately cool the hair removal apparatus 30 and the heat conduction part 50 through the cooling channel 22. In this way, an internal structure of the hair removal instrument is optimized, and heat dissipation efficiency inside the hair removal instrument is improved. The cooling driver 10 drives the external cooling medium to flow, so that the external cooling medium can effectively take away heat of the hair removal instrument in the double-duct channel.

The heat conduction part 50 includes a heat conduction member 51 and a graphite sheet 52 made of a graphene material. The graphite sheet 52 is fastened on the heat conduction member 51. The graphite sheet 52 made of the graphene material may enhance heat conduction efficiency of the heat conduction member 51. Heat conduction performance of the heat conduction part 50 may be significantly improved compared with that of a conventional heat conduction part. One end of the heat conduction member 51 is located in the cooling channel 22, and the other end of the heat conduction member 51 is connected to the cold compress part 40.

The heat conduction member 51 includes a vapor chamber 511 connected to the cold compress part 40 and several heat sinks 512 fastened on a bottom part of the vapor chamber 511. The graphite sheet 52 is fastened on a top part of the vapor chamber 511, the vapor chamber 511 is fastened on the inner wall of the housing 20, one end that is of the vapor chamber 511 and that is away from the cold compress part 40 covers the second cooling duct 222, the heat sinks 512 are accommodated in the second cooling duct 222, the heat sinks 512 contact a part that is of the vapor chamber 511 and that covers the second cooling duct 222, and the heat sinks 512 can improve a heat dissipation effect.

The hair removal instrument generates heat during operation. The cooling driver 10 can drive the external cooling medium to flow into the housing 20. The external cooling medium is used to take away the heat of the heat conduction part 50, to quickly cool devices inside the hair removal instrument, thereby avoiding excessive heat in an internal space of the hair removal instrument, and preventing devices in the hair removal apparatus 30 from undergoing dangerous situations such as a short circuit, burning, and explosion.

In this embodiment, the cooling driver 10 is a turbo fan, the cooling medium is preferably cooling air, the housing 20 is further provided with an air inlet 251, and the air inlet 251 is disposed corresponding to the cooling driver 10. The cooling channel 22 is located inside the housing 20, the air inlet 251 is in communication with the air outlet 21 through the driving port 11 and the cooling channel 22, and the air inlet 251 is disposed on an edge of the housing 20.

The cooling driver 10 has a guide port 13. One end of the guide port 13 is in communication with the driving port 11, and the other end of the guide port 13 is disposed corresponding to the air inlet 251 and is in communication with the air inlet 251. The hair removal instrument further includes an elastic sealing gasket 80. The elastic sealing gasket 80 has a penetrating hole 81. A gap is disposed between the housing 20 and one surface that is of the turbo fan and that faces the air inlet 251. The elastic sealing gasket 80 is clamped between the turbo fan and the housing 20 to block the gap. The air inlet 251, the penetrating hole 81, and the guide port 13 are sequentially communicated, to prevent a heat dissipation effect of the turbo fan from being poor because hot air in the housing 20 is sucked into the turbo fan again. Certainly, a situation in which hot air coming from the air outlet 21 is sucked into the cooling driver 10 again is an exception.

In some embodiments, an inner diameter of the penetrating hole 81 is greater than an inner diameter of the air inlet 251, and the air inlet 251, the penetrating hole 81, and the guide port 13 are coaxially disposed, so that external air can smoothly enter the guide port 13.

The housing 20 includes a bottom housing 24, a top housing 25 covering the bottom housing 24, and a separator 26 configured to separate the cooling channel 22 into the first cooling duct 221 and the second cooling duct 222. All of the separator 26, the cooling driver 10, the control board 60, the hair removal apparatus 30, the cold compress part 40, the heat conduction part 50, and the temperature control apparatus 70 are clamped between the bottom housing 24 and the top housing 25. The air inlet 251 is disposed corresponding to the cooling driver 10, and the air inlet 251 is in communication with the air outlet 21 through the driving port 11. The reflector cup 33 is mounted on the separator 26.

In some embodiments, the reflector cup 33 is provided with a first U-shaped groove 331 in a length direction of the reflector cup 33, and the length direction of the reflector cup 33 is consistent with a flow direction of the cooling medium. The filament-free lamp tube 31 is located in the first U-shaped groove 331 and abuts against an inner wall of the first U-shaped groove 331, so that the light of the filament-free lamp tube 31 is reflected and focused on the cold compress part 40.

The reflector cup 33 is of a U-shaped structure, and the separator 26 is provided with a second U-shaped groove 261 in a length direction of the separator 26 and several clamping grooves 262 recessed on an inner wall of the second U-shaped groove 261. The second U-shaped groove 261 is located on one side that is of the separator 26 and that faces the cold compress part 40, and the control board 60 is fastened on the bottom housing 24. Several clamping blocks 332 protrude from the reflector cup 33, the clamping blocks 332 are clamped in the clamping grooves 262, and a part of the reflector cup 33 is located in the second U-shaped groove 261, to prevent a foreign object from colliding with the reflector cup 33.

The housing 20 further includes a cover plate 27. The cover plate 27 is mounted on the housing 20 and is located at the air inlet 251, the cover plate 27 is provided with several air intake holes 271 in communication with the guide port 13, and the cover plate 27 may protect the cooling driver 10 from hurting people.

The hair removal instrument further includes a sealing cover 28. One end of the sealing cover 28 is mounted on the air outlet 21 of the housing 20 and seals the air outlet 21, and the other end of the sealing cover 28 is connected to the heat conduction part 50. The sealing cover 28 is provided with air ventilation holes 281 and an air guide hole 282. One end of the air guide hole 282 is in communication with the cooling channel 22, and the other end of the air guide hole 282 is in communication with the air ventilation holes 281. The air guide hole 282 is configured to concentrate hot air coming from the cooling channel 22, so that the hot air is blown to the air ventilation holes 281 by using the cooling medium and is dissipated from the air ventilation holes 281. In this way, the sealing cover 28 implements concentrated air exhaust.

The temperature control apparatus 70 is mounted on the sealing cover 28 and is located in the air guide hole 282. Preferably, the sealing cover 28 includes an air guide housing 283 and a sealing plate 284. The air guide housing 283 is annular, one end of the air guide housing 283 is mounted in the air outlet 21, and the other end of the air guide housing 283 is connected to the heat conduction part 50. The air guide hole 282 penetrates through the air guide housing 283. Both the sealing plate 284 and the temperature control apparatus 70 are mounted on the air guide housing 283. The sealing plate 284 is configured to seal the air guide hole 282. The air ventilation holes 281 penetrate through the sealing plate 284. The air guide housing 283 fits with the sealing plate 284 to guide the hot air out of the housing 20. This prevents hot air backflow, and can improve both heat dissipation efficiency and a heat dissipation effect. The temperature control apparatus 70 is located between the sealing plate 284 and the heat conduction part 50. The temperature control apparatus 70 is mounted on an independent sealing cover 28. The temperature control apparatus 70 is configured to detect a temperature of hot air in the air guide hole 282. When the temperature control apparatus 70 detects that the temperature at the air guide hole 282 is excessively high, the temperature control apparatus 70 transmits information to the control board 60, and the control board 60 powers off the hair removal apparatus 30, so that the hair removal apparatus 30 stops operating, thereby avoiding a safety hazard caused by an excessively high temperature inside the housing 20.

In this embodiment, the cooling channel 22 includes the first cooling duct 221 and the second cooling duct 222. The sealing cover 28 concentrates hot air coming from the first cooling duct 221 and the second cooling duct 222, and guides the hot air to the air guide hole 282 and the air ventilation holes 281, so that the blown hot air can be isolated from an inner cavity of the housing 20, thereby preventing the hot air from reentering the housing 20 after dissipation. In addition, the sealing cover 28 can prevent the temperature control apparatus 70 from collision.

The hair removal instrument further includes a capacitor 90. The capacitor 90 is fastened in the housing 20, the capacitor 90 is electrically connected to the control board 60, and the capacitor 90 may be used as a part of the control board 60 to store power.

In some embodiments, the hair removal instrument further includes a power adapter 100. The power adapter 100 is located outside the housing 20, and the power adapter 100 is connected to the control board 60 by using a wire 110, to convert an external alternating current into a direct current required by the devices inside the hair removal instrument.

The cold compress part 40 includes a cold compress plate 41 configured to adhere to skin and a refrigeration member 42 connected to the cold compress plate 41. The cold compress plate 41 is fastened on the light outlet 23 and is connected to the heat conduction part 50. Heat of the cold compress part 40 is transmitted from the cold compress plate 41 to the heat conduction part 50, so that the cold compress part 40 achieves a refrigeration effect, and a cooling rate is accelerated. The refrigeration member 42 is electrically connected to the control board 60.

The hair removal instrument further includes a start button 200 configured to control power-on of the hair removal apparatus 30. An embedding hole further penetrates through the housing 20, and the start button 200 is mounted on the embedding hole and is electrically connected to the control board 60, to control on/off of the filament-free lamp tube 31.

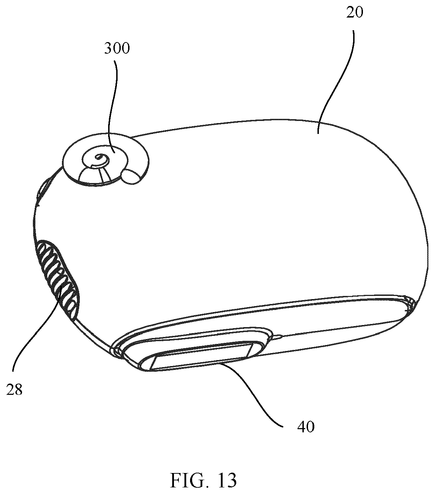

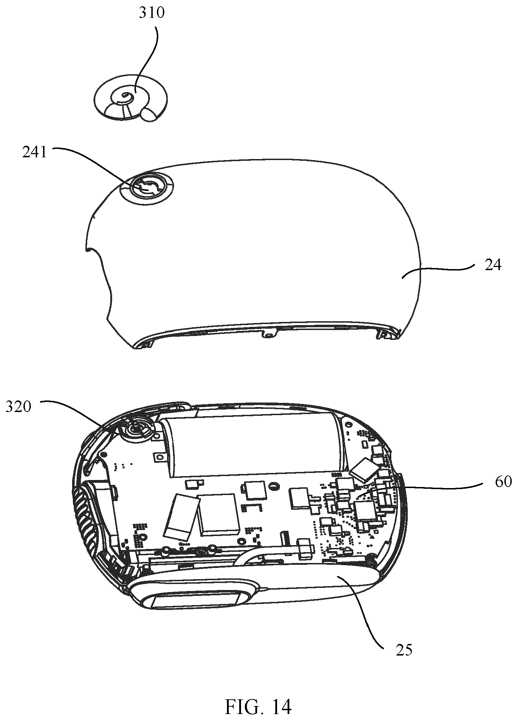

Further, refer to FIG. 13 and FIG. 14. The hair removal instrument further includes a regulator 300 configured to regulate a power of the filament-free lamp tube 31. A mounting hole 241 further penetrates through the housing 20, and the regulator 300 is mounted on the mounting hole 241 and is electrically connected to the control board 60, to regulate brightness of the filament-free lamp tube 31 at any time.

The regulator 300 includes a rotary knob 310 and a gear switch 320 mounted on the control board 60. The gear switch 320 is a rotary switch with a torsion spring inside. The rotary knob 310 is mounted on the mounting hole 241 and is rotatably connected to the gear switch 320. Power levels of the filament-free lamp tube 31 may be classified into three levels. Each time the rotary knob 310 rotates, the brightness of the filament-free lamp tube 31 changes once. The three power levels may be cycled sequentially. The rotary knob 310 returns to an initial state after each time of rotation, and the rotary knob 310 is of an ear-shaped rotary structure with a mechanical sense when being touched.

The hair removal instrument further includes a trigger pulse button 400 configured to provide the filament-free lamp tube 31 with an instantaneous high-voltage pulse. A clamping hole further penetrates through the housing 20, and the trigger pulse button 400 is mounted on the clamping hole and is electrically connected to the control board 60. The filament-free lamp tube 31 may be provided with the instantaneous high-voltage pulse when the trigger pulse button 400 is pressed. In this case, the filament-free lamp tube 31 is triggered and controlled to light up to generate the laser light, so that the hair removal instrument operates. The filament-free lamp tube 31 does not light up when the trigger pulse button 400 is not pressed.

In addition, there is a display lamp around the trigger pulse button 400 for instruction. The display lamp needs to contact the skin to be displayed. In this case, the trigger pulse button 400 can be pressed for operation. If the display lamp does not contact the skin, the display lamp is not displayed. In this case, the trigger pulse button 400 does not operate even if the trigger pulse button 400 is pressed.

Compared with the conventional technology, the hair removal instrument in this application has the following advantages:
1. In this application, the sealing cover 28 is mounted on the air outlet 21 of the housing 20, and the sealing cover 28 is configured to seal the air outlet 21. The sealing cover 28 is provided with the air ventilation holes 281 and the air guide hole 282, one end of the air guide hole 282 is in communication with the cooling channel 22, and the other end of the air guide hole 282 is in communication with the air ventilation holes 281. The air guide hole 282 is configured to concentrate the hot air coming from the cooling channel 22, so that air exhaust is performed at the air ventilation holes 281 under driving of the cooling medium. In this way, the sealing cover 28 implements concentrated air exhaust.
2. The hair removal instrument is additionally provided with the temperature control apparatus 70. The temperature control apparatus 70 is disposed in the air guide hole 282. The cooling driver 10 may output the cooling medium through the driving port 11. The cooling medium is preferably cooling air. The cooling medium may be used to dissipate the heat on the heat conduction part 50 from the air ventilation holes 281. When the temperature control apparatus 70 detects that the temperature at the air ventilation holes 281 is excessively high, the temperature control apparatus 70 transmits the information to the control board 60, and the control board 60 controls the hair removal apparatus 30 to stop operating, thereby avoiding the safety hazard caused by the excessively high temperature inside the housing 20.
3. The regulator 300 is additionally provided, and the regulator 300 may be configured to regulate the power of the filament-free lamp tube 31 to change the brightness of the filament-free lamp tube 31.
4. The graphite sheet 52 is added above the vapor chamber 511. The graphite sheet 52 made of the graphene material can enhance the heat conduction efficiency, to improve the heat dissipation effect of the heat conduction part 50.
5. The reflector cup 33 is electrically connected to the control board 60 by using the elastic conductive member 61. The elastic conductive member 61 may be a spring sheet or an assembly including a spring. The elastic conductive member 61 has the contact pressure with the reflector cup 33 on the premise of being electrically connected to the control board 60. This ensures the connection effectiveness, the solid structure, and the high stability. In addition, the elastic conductive member 61 improves the assembly and maintenance efficiency of the hair removal instrument.
6. The elastic sealing gasket 80 is additionally provided. The gap is disposed between the housing 20 and the surface that is of the turbo fan and that faces the air inlet 251. The elastic sealing gasket 80 is clamped between the turbo fan and the housing 20 to block the gap. The air inlet 251, the penetrating hole 81, and the guide port 13 are sequentially communicated, to prevent the heat dissipation effect of the turbo fan from being poor because the hot air in the housing 20 is sucked into the turbo fan again.

The foregoing descriptions are merely some embodiments of this application, and are not intended to limit the protection scope of this application. Any equivalent apparatus or equivalent process transformation that is made according to the content in the specification and the accompanying drawings of this application and that is directly or indirectly applied to another related technical field is similarly included in the patent protection scope of this application.

## Claims

1. A hair removal instrument, comprising:
a cooling driver (10), provided with a driving port (11), wherein the cooling driver (10) is capable of outputting a cooling medium through the driving port (11);
a housing (20), wherein the cooling driver (10) is fastened in the housing (20), and the housing is provided with an air outlet (21), wherein the housing (20) comprises a cooling channel (22) butt-jointed to the driving port (11) and in communication with the air outlet (21), and a light outlet (23) in communication with the cooling channel (22);
a hair removal apparatus (30), located in the housing (20) and fastened in the cooling channel (22);
a cold compress part (40), fastened on the light outlet (23) and opposite to the hair removal apparatus (40), configured to receive light emitted by the hair removal apparatus (30) and emit the light;
a heat conduction part (50), fastened in the housing (20), wherein one end of the heat conduction part (50) is located in the cooling channel (22), and the other end of the heat conduction part (50) is connected to the cold compress part (40), wherein the cooling medium in the cooling channel (22) is also used to dissipate the heat on the heat conduction portion (50) from the air outlet (21), so that heat in the housing (20) is reduced;
a temperature control apparatus (70), mounted in the housing (20) and located on one side of the heat conduction part (50), the aforesaid side being located away from the cooling driver (10) and close to the air outlet (21);
a control board (60), fastened in the housing (20), wherein all of the cooling driver (10), the hair removal apparatus (30), and the temperature control apparatus (70) are electrically connected to the control board (60);
wherein the temperature control apparatus (70) is configured to detect the temperature at the air outlet (21), and wherein the control board (60) is configured to control the operation of the hair removal apparatus (30) according to the temperature detected by the temperature control device (70).

2. The hair removal instrument according to claim 1, comprising a sealing cover (28), wherein one end of the sealing cover (28) is fastened on the air outlet (21) and seals the air outlet (21), and the other end of the sealing cover (28) is connected to the heat conduction part (50), wherein the sealing cover is provided with an air guide hole (282) and several air ventilation holes (281), wherein one end of the air guide hole (282) is in communication with the cooling channel (22), the other end of the air guide hole (282) is in communication with the air ventilation holes (281), and the air guide hole (282) is configured to concentrate hot air coming from the cooling channel (22), so that the hot air is dissipated from the air ventilation holes by using the cooling medium;
wherein the temperature control apparatus (70) is located on an inner wall of the air guide hole (282).

3. The hair removal instrument according to claim 1, wherein the hair removal apparatus comprises a filament-free lamp tube (31), a lamp holder (32) fastened on the control board, and a reflector cup (33) made of an electrically conductive material, wherein both the reflector cup and the lamp holder are located in the cooling channel, the reflector cup is detachably mounted on the housing and is electrically connected to the control board, the filament-free lamp tube is mounted on the lamp holder and abuts against the reflector cup, wherein
the reflector cup is located on one side that is of the filament-free lamp tube and that is located away from the cold compress part, wherein the reflector cup is configured to reflect and focus light of the filament-free lamp tube on the cold compress part.

4. The hair removal instrument according to claim 3, wherein an elastic conductive member (61) electrically connected to the control board is fastened on the control board, and the reflector cup is pressed against the elastic conductive member to be electrically connected to the control board by using the elastic conductive member.

5. The hair removal instrument according to claim 3, further comprising a regulator (300) configured to regulate a power of the filament-free lamp tube, wherein a mounting hole (241) penetrates through the housing (20), and the regulator is mounted on the mounting hole and is electrically connected to the control board.

6. The hair removal instrument according to claim 5, wherein the regulator comprises an ear-shaped rotary knob (310) and a gear switch (320) mounted on the control board, and the rotary knob is mounted on the mounting hole and is rotatably connected to the gear switch.

7. The hair removal instrument according to claim 3, further comprising a trigger pulse button (400) configured to provide the filament-free lamp tube with an instantaneous high-voltage pulse, wherein a clamping hole penetrates through the housing, and wherein the trigger pulse button is mounted on the clamping hole and is electrically connected to the control board.

8. The hair removal instrument according to claim 3, wherein the cooling channel comprises a first cooling duct (221) and a second cooling duct (222), wherein the first cooling duct and the second cooling duct are disposed in parallel, one end of the first cooling duct and one end of the second cooling duct are butt-jointed to the driving port, the other end of the first cooling duct and the other end of the second cooling duct are butt-jointed to the air outlet, wherein
the hair removal apparatus is located in the first cooling duct, a part of the heat conduction part is located in the second cooling duct, a baffle plate (12) configured to limit a capacity of a cooling medium entering the first cooling duct is obliquely disposed in the driving port, and the cooling medium is capable of taking away heat on the heat conduction part.

9. The hair removal instrument according to claim 8, wherein the heat conduction part comprises a heat conduction member (51) and a graphite sheet (52) made of a graphene material, wherein
the graphite sheet is fastened on the heat conduction member, one end of the heat conduction member is located in the cooling channel, and the other end of the heat conduction member is connected to the cold compress part.

10. The hair removal instrument according to claim 9, wherein the heat conduction member comprises a vapor chamber (511) connected to the cold compress part and several heat sinks (512) fastened on a bottom part of the vapor chamber, wherein the graphite sheet is fastened on a top part of the vapor chamber, the vapor chamber is fastened to an inner wall of the housing, one end that is of the vapor chamber and that is away from the cold compress part covers the second cooling duct, and the heat sinks are accommodated in the second cooling duct and contact a part that is of the vapor chamber and that covers the second cooling duct.

11. The hair removal instrument according to claim 8, wherein the housing comprises a bottom housing (24), a top housing (25) covering the bottom housing, and a separator (26) configured to separate the cooling channel into the first cooling duct and the second cooling duct, wherein all of the separator, the cooling driver, the control board, the hair removal apparatus, the cold compress part, the heat conduction part, and the temperature control apparatus are clamped between the bottom housing and the top housing.

12. The hair removal instrument according to claim 11, wherein the reflector cup is mounted on the separator;
wherein the reflector cup is provided with a first U-shaped groove (331) in a length direction of the reflector cup, wherein the length direction of the reflector cup is consistent with a flow direction of the cooling medium, and wherein the filament-free lamp tube is located in the first U-shaped groove and abuts against an inner wall of the first U-shaped groove.

13. The hair removal instrument according to claim 1, wherein the cooling driver is a turbo fan, the housing is further provided with an air inlet (251), the air inlet is disposed corresponding to the cooling driver, the cooling channel is located inside the housing, the air inlet is in communication with the air outlet through the driving port and the cooling channel, and all of the air inlet, the air outlet, and the light outlet are disposed on an edge of the housing.

14. The hair removal instrument according to claim 1, wherein the cooling driver further has a guide port (13), wherein the guide port is separately in communication with the air inlet and the driving port;
wherein the hair removal instrument further comprises an elastic sealing gasket (80), wherein the elastic sealing gasket has a penetrating hole (81), a gap is disposed between the housing and one surface of the cooling driver, and wherein the aforesaid surface faces the air inlet;
wherein the elastic sealing gasket is clamped between the cooling driver and the housing to block the gap, and the air inlet, the penetrating hole, and the guide port are sequentially communicated.

15. The hair removal instrument according to claim 1, further comprising a power adapter (100), wherein the power adapter is located in the housing, and the power adapter is electrically connected to the control board by using a wire (110); and/or
wherein the hair removal instrument further comprises a start button (200) configured to control power-on of the hair removal apparatus, wherein an embedding hole penetrates through the housing, and wherein the start button is mounted on the embedding hole and is electrically connected to the control board.

## Patentansprüche

1. Haarentfernungsgerät, umfassend:
einen Kühltreiber (10), der mit einem Antriebsanschluss (11) versehen ist, wobei der Kühltreiber (10) in der Lage ist, ein Kühlmedium durch den Antriebsanschluss (11) auszugeben;
ein Gehäuse (20), wobei der Kühltreiber (10) in dem Gehäuse (20) befestigt ist und das Gehäuse mit einem Luftauslass (21) versehen ist, wobei das Gehäuse (20) einen Kühlkanal (22), der sich an den Antriebsanschluss (11) anschließt und mit dem Luftauslass (21) in Verbindung steht, und einen Lichtauslass (23), der mit dem Kühlkanal (22) in Verbindung steht, umfasst;
eine Haarentfernungsvorrichtung (30), die sich im Gehäuse (20) befindet und im Kühlkanal (22) befestigt ist;
ein Kaltkompressenteil (40), das am Lichtauslass (23) befestigt ist und der Haarentfernungsvorrichtung (30) gegenüberliegt und dazu konfiguriert ist, das von der Haarentfernungsvorrichtung (30) emittierte Licht zu empfangen und auszusenden;
ein Wärmeleitungsteil (50), das in dem Gehäuse (20) befestigt ist, wobei ein Ende des Wärmeleitungsteils (50) in dem Kühlkanal (22) angeordnet ist und das andere Ende des Wärmeleitungsteils (50) mit dem Kaltkompressenteil (40) verbunden ist, wobei das Kühlmedium in dem Kühlkanal (22) ferner dazu verwendet wird, die Wärme an dem Wärmeleitungsteil (50) von dem Luftauslass (21) abzuführen, so dass die Wärme in dem Gehäuse (20) reduziert wird;
eine Temperatursteuervorrichtung (70), die in dem Gehäuse (20) angebracht und auf einer Seite des Wärmeleitungsteils (50) angeordnet ist, wobei die vorgenannte Seite von dem Kühltreiber (10) abgewandt und nahe dem Luftauslass (21) angeordnet ist;
eine Steuerplatine (60), die in dem Gehäuse (20) befestigt ist, wobei der Kühltreiber (10), die Haarentfernungsvorrichtung (30) und die Temperatursteuervorrichtung (70) alle mit der Steuerplatine (60) elektrisch verbunden sind;
wobei die Temperatursteuervorrichtung (70) dazu konfiguriert ist, die Temperatur am Luftauslass (21) zu erfassen, und wobei die Steuerplatine (60) dazu konfiguriert ist, den Betrieb der Haarentfernungsvorrichtung (30) entsprechend der von der Temperatursteuervorrichtung (70) erfassten Temperatur zu steuern.

2. Haarentfernungsgerät nach Anspruch 1, umfassend eine Dichtungsabdeckung (28), wobei ein Ende der Dichtungsabdeckung (28) am Luftauslass (21) befestigt ist und den Luftauslass (21) abdichtet, und das andere Ende der Dichtungsabdeckung (28) mit dem Wärmeleitungsteil (50) verbunden ist, wobei die Dichtungsabdeckung mit einem Luftführungsloch (282) und mehreren Belüftungslöchern (281) versehen ist, wobei
ein Ende des Luftführungslochs (282) mit dem Kühlkanal (22) in Verbindung steht, das andere Ende des Luftführungslochs (282) mit den Belüftungslöchern (281) in Verbindung steht und das Luftführungsloch (282) dazu konfiguriert ist, vom Kühlkanal (22) kommende Heißluft zu konzentrieren, so dass die Heißluft aus den Belüftungslöchern (281) unter Verwendung des Kühlmediums abgeführt wird;
wobei die Temperatursteuervorrichtung (70) an einer Innenwand des Luftführungslochs (282) angeordnet ist.

3. Haarentfernungsgerät nach Anspruch 1, wobei die Haarentfernungsvorrichtung (30) eine glühfadenfreie Lampenröhre (31), eine an der Steuerplatine (60) befestigte Lampenhalterung (32) und einen aus einem elektrisch leitfähigen Material hergestellten Reflektorbecher (33) umfasst, wobei sowohl der Reflektorbecher als auch die Lampenhalterung in dem Kühlkanal angeordnet sind, der Reflektorbecher abnehmbar an dem Gehäuse angebracht ist und mit der Steuerplatine elektrisch verbunden ist, die glühfadenfreie Lampenröhre an der Lampenhalterung angebracht ist und an dem Reflektorbecher anliegt, wobei
der Reflektorbecher auf einer Seite der glühfadenfreien Lampenröhre angeordnet ist, die von dem Kaltkompressenteil abgewandt ist, wobei
der Reflektorbecher dazu konfiguriert ist, das Licht der glühfadenfreien Lampenröhre zu reflektieren und auf das Kaltkompressenteil zu fokussieren.

4. Haarentfernungsgerät nach Anspruch 3, wobei ein elastisches, leitfähiges Element (61), das mit der Steuerplatine elektrisch verbunden ist, an der Steuerplatine befestigt ist, und der Reflektorbecher gegen das elastische, leitfähige Element gedrückt wird, um durch Verwendung des elastischen, leitfähigen Elements mit der Steuerplatine elektrisch verbunden zu werden.

5. Haarentfernungsgerät nach Anspruch 3, ferner umfassend einen Regler (300), der dazu konfiguriert ist, eine Leistung der glühfadenfreien Lampenröhre zu regeln, wobei
ein Montageloch (241) das Gehäuse (20) durchdringt und der Regler an dem Montageloch angebracht und mit der Steuerplatine elektrisch verbunden ist.

6. Haarentfernungsgerät nach Anspruch 5, wobei der Regler einen ohrförmigen Drehknopf (310) und einen Getriebeschalter (320), der an der Steuerplatine angebracht ist, umfasst, und der Drehknopf an dem Montageloch angebracht ist und drehbar mit dem Getriebeschalter verbunden ist.

7. Haarentfernungsgerät nach Anspruch 3, ferner umfassend eine Auslöseimpulstaste (400), die dazu konfiguriert ist, die glühfadenfreie Lampenröhre mit einem sofortigen Hochspannungsimpuls zu versorgen, wobei
ein Klemmloch das Gehäuse durchdringt, und wobei
die Auslöseimpulstaste an dem Klemmloch angebracht ist und mit der Steuerplatine elektrisch verbunden ist.

8. Haarentfernungsgerät nach Anspruch 3, wobei der Kühlkanal einen ersten Kühlschlauch (221) und einen zweiten Kühlschlauch (222) umfasst, wobei
der erste Kühlschlauch und der zweite Kühlschlauch parallel angeordnet sind, ein Ende des ersten Kühlschlauchs und ein Ende des zweiten Kühlschlauchs sich an den Antriebsanschluss anschließen, das andere Ende des ersten Kühlschlauchs und das andere Ende des zweiten Kühlschlauchs sich an den Luftauslass anschließen, wobei
die Haarentfernungsvorrichtung in dem ersten Kühlschlauch angeordnet ist, ein Teil des Wärmeleitungsteils in dem zweiten Kühlschlauch angeordnet ist, eine Anschlagplatte (12), die dazu konfiguriert ist, eine Kapazität eines Kühlmediums, das in den ersten Kühlschlauch eintritt, zu begrenzen, schräg in dem Antriebsanschluss angeordnet ist, und das Kühlmedium in der Lage ist, Wärme an dem Wärmeleitungsteil abzuführen.

9. Haarentfernungsgerät nach Anspruch 8, wobei das Wärmeleitungsteil ein Wärmeleitungselement (51) und ein Graphitblatt (52) aus einem Graphenmaterial umfasst, wobei
das Graphitblatt an dem Wärmeleitungselement befestigt ist, ein Ende des Wärmeleitungselements in dem Kühlkanal angeordnet ist und das andere Ende des Wärmeleitungselements mit dem Kaltkompressenteil verbunden ist.

10. Haarentfernungsgerät nach Anspruch 9, wobei das Wärmeleitungselement eine Dampfkammer (511), die mit dem Kaltkompressenteil verbunden ist, und mehrere Wärmesenken (512), die an einem unteren Teil der Dampfkammer befestigt sind, umfasst, wobei
das Graphitblatt an einem oberen Teil der Dampfkammer befestigt ist, die Dampfkammer an einer Innenwand des Gehäuses befestigt ist, ein Ende der Dampfkammer, das von dem Kaltkompressenteil abgewandt ist, den zweiten Kühlschlauch abdeckt, und die Wärmesenken in dem zweiten Kühlschlauch untergebracht sind und mit einem Teil der Dampfkammer in Kontakt stehen, das den zweiten Kühlschlauch abdeckt.

11. Haarentfernungsgerät nach Anspruch 8, wobei das Gehäuse ein Untergehäuse (24), ein Obergehäuse (25), das das Untergehäuse abdeckt, und einen Separator (26) umfasst, der dazu konfiguriert ist, den Kühlkanal in den ersten Kühlschlauch und den zweiten Kühlschlauch zu trennen, wobei
der Separator, der Kühltreiber, die Steuerplatine, die Haarentfernungsvorrichtung, das Kaltkompressenteil, das Wärmeleitungsteil und die Temperatursteuervorrichtung alle zwischen dem Untergehäuse und dem Obergehäuse eingeklemmt sind.

12. Haarentfernungsgerät nach Anspruch 11, wobei der Reflektorbecher an dem Separator angebracht ist;
wobei der Reflektorbecher mit einer ersten U-förmigen Nut (331) in einer Längsrichtung des Reflektorbechers versehen ist, wobei
die Längsrichtung des Reflektorbechers mit der Strömungsrichtung des Kühlmediums übereinstimmt, und wobei
die glühfadenfreie Lampenröhre in der ersten U-förmigen Nut angeordnet ist und an einer Innenwand der ersten U-förmigen Nut anliegt.

13. Haarentfernungsgerät nach Anspruch 1, wobei der Kühltreiber ein Turbolüfter ist, das Gehäuse ferner mit einem Lufteinlass (251) versehen ist, der Lufteinlass entsprechend dem Kühltreiber angeordnet ist, der Kühlkanal innerhalb des Gehäuses angeordnet ist, der Lufteinlass über den Antriebsanschluss und den Kühlkanal mit dem Luftauslass in Verbindung steht und der Lufteinlass, der Luftauslass und der Lichtauslass alle an einem Rand des Gehäuses angeordnet sind.

14. Haarentfernungsgerät nach Anspruch 1, wobei der Kühltreiber ferner einen Führungsanschluss (13) aufweist, wobei
der Führungsanschluss separat mit dem Lufteinlass und dem Antriebsanschluss in Verbindung steht;
wobei das Haarentfernungsgerät ferner eine elastische Dichtung (80) umfasst, wobei
die elastische Dichtung eine Durchgangsöffnung (81) aufweist, ein Spalt zwischen dem Gehäuse und einer Fläche des Kühltreibers vorhanden ist, und wobei
die vorgenannte Fläche dem Lufteinlass zugewandt ist;
wobei die elastische Dichtung zwischen dem Kühltreiber und dem Gehäuse eingeklemmt ist, um den Spalt zu schließen, und der Lufteinlass, die Durchgangsöffnung und der Führungsanschluss nacheinander in Verbindung stehen.

15. Haarentfernungsgerät nach Anspruch 1, ferner umfassend einen Stromadapter (100), wobei
der Stromadapter in dem Gehäuse angeordnet ist und der Stromadapter mit der Steuerplatine unter Verwendung eines Kabels (110) elektrisch verbunden ist; und/oder
wobei das Haarentfernungsgerät ferner eine Starttaste (200) umfasst, die dazu konfiguriert ist, das Einschalten der Haarentfernungsvorrichtung zu steuern, wobei
ein Einbettungsloch das Gehäuse durchdringt, und wobei
die Starttaste an dem Einbettungsloch angebracht ist und mit der Steuerplatine elektrisch verbunden ist.

## Revendications

1. Épilateur, comprenant :
un pilote de refroidissement (10), pourvu d'un orifice d'entraînement (11), dans lequel le pilote de refroidissement (10) est capable de fournir un fluide de refroidissement à travers l'orifice d'entraînement (11) ;
un boîtier (20), dans lequel le pilote de refroidissement (10) est fixé dans le boîtier (20), et le boîtier est pourvu d'une sortie d'air (21), dans lequel le boîtier (20) comprend un canal de refroidissement (22) relié bout à bout à l'orifice d'entraînement (11) et en communication avec la sortie d'air (21), et une sortie de lumière (23) en communication avec le canal de refroidissement (22) ;
un composant épilatoire (30), situé dans le boîtier (20) et fixé dans le canal de refroidissement (22) ;
une portion de compresse froide (40), fixée sur la sortie de lumière (23) et à l'opposé du composant épilatoire (30), configurée pour recevoir une lumière émise par le composant épilatoire (30) et émettre la lumière ;
une portion de conduction thermique (50), fixée dans le boîtier (20), dans laquelle une extrémité de la portion de conduction thermique (50) est située dans le canal de refroidissement (22), et l'autre extrémité de la portion de conduction thermique (50) est reliée à la portion de compresse froide (40), dans laquelle le fluide de refroidissement dans le canal de refroidissement (22) est également utilisé pour dissiper la chaleur sur la portion de conduction thermique (50) depuis la sortie d'air (21), de sorte que la chaleur dans le boîtier (20) soit réduite ;
un composant de régulation de température (70), monté dans le boîtier (20) et situé sur un côté de la portion de conduction thermique (50), ledit côté étant situé à l'écart du pilote de refroidissement (10) et proche de la sortie d'air (21) ;
un tableau de commande (60), fixé dans le boîtier (20), dans lequel le pilote de refroidissement (10), le composant épilatoire (30) et le composant de régulation de température (70) sont tous connectés électriquement au tableau de commande (60) ;
dans lequel le composant de régulation de température (70) est configuré pour détecter la température au niveau de la sortie d'air (21), et dans lequel le tableau de commande (60) est configuré pour commander le fonctionnement du composant épilatoire (30) en fonction de la température détectée par le composant de régulation de température (70).

2. Épilateur selon la revendication 1, comprenant un couvercle d'étanchéité (28), dans lequel une extrémité du couvercle d'étanchéité (28) est fixée sur la sortie d'air (21) et étanche la sortie d'air (21), et l'autre extrémité du couvercle d'étanchéité (28) est reliée à la portion de conduction thermique (50), dans lequel le couvercle d'étanchéité est pourvu d'un trou de guidage d'air (282) et de plusieurs trous de ventilation d'air (281), dans lequel
une extrémité du trou de guidage d'air (282) est en communication avec le canal de refroidissement (22), l'autre extrémité du trou de guidage d'air (282) est en communication avec les trous de ventilation d'air (281), et le trou de guidage d'air (282) est configuré pour concentrer un air chaud provenant du canal de refroidissement (22), de sorte que l'air chaud soit dissipé depuis les trous de ventilation d'air (281) par l'intermédiaire du fluide de refroidissement ;
dans lequel le composant de régulation de température (70) est situé sur une paroi interne du trou de guidage d'air (282).

3. Épilateur selon la revendication 1, dans lequel le composant épilatoire (30) comprend un tube de lampe sans filament (31), une porte-lampe (32) fixée sur le tableau de commande (60) et une coupelle réflectrice (33) en matériau électriquement conducteur, dans lequel la coupelle réflectrice et la porte-lampe sont toutes deux situées dans le canal de refroidissement, la coupelle réflectrice est montée de manière amovible sur le boîtier et est connectée électriquement au tableau de commande, et le tube de lampe sans filament est monté sur la porte-lampe et s'appuie contre la coupelle réflectrice, dans lequel
la coupelle réflectrice est située sur un côté du tube de lampe sans filament à l'écart de la portion de compresse froide, dans laquelle
la coupelle réflectrice est configurée pour réfléchir et focaliser une lumière du tube de lampe sans filament sur la portion de compresse froide.

4. Épilateur selon la revendication 3, dans lequel un élément conducteur élastique (61) connecté électriquement au tableau de commande est fixé sur le tableau de commande, et la coupelle réflectrice s'appuie contre l'élément conducteur élastique pour être connectée électriquement au tableau de commande par l'intermédiaire de l'élément conducteur élastique.

5. Épilateur selon la revendication 3, comprenant en outre un régulateur (300) configuré pour réguler une puissance du tube de lampe sans filament, dans lequel
un trou de montage (241) pénètre à travers le boîtier (20), et le régulateur est monté sur le trou de montage et est connecté électriquement au tableau de commande.

6. Épilateur selon la revendication 5, dans lequel le régulateur comprend un bouton rotatif en forme d'oreille (310) et un interrupteur à engrenage (320) monté sur le tableau de commande, et le bouton rotatif est monté sur le trou de montage et est relié de manière rotative à l'interrupteur à engrenage.

7. Épilateur selon la revendication 3, comprenant en outre un bouton d'impulsion de déclenchement (400) configuré pour fournir une impulsion instantanée à haute tension au tube de lampe sans filament, dans lequel
un trou de serrage pénètre à travers le boîtier, et dans lequel
le bouton d'impulsion de déclenchement est monté sur le trou de serrage et est connecté électriquement au tableau de commande.

8. Épilateur selon la revendication 3, dans lequel le canal de refroidissement comprend un premier conduit de refroidissement (221) et un second conduit de refroidissement (222), dans lequel
le premier conduit de refroidissement et le second conduit de refroidissement sont disposés en parallèle, une extrémité du premier conduit de refroidissement et une extrémité du second conduit de refroidissement sont reliées bout à bout à l'orifice d'entraînement, et l'autre extrémité du premier conduit de refroidissement et l'autre extrémité du second conduit de refroidissement sont reliées bout à bout à la sortie d'air, dans lequel
le composant épilatoire est situé dans le premier conduit de refroidissement, une partie de la portion de conduction thermique est située dans le second conduit de refroidissement, une plaque de déflecteur (12) configurée pour limiter une capacité d'un fluide de refroidissement entrant dans le premier conduit de refroidissement est disposée obliquement dans l'orifice d'entraînement, et le fluide de refroidissement est capable de dissiper la chaleur sur la portion de conduction thermique.

9. Épilateur selon la revendication 8, dans lequel la portion de conduction thermique comprend un élément de conduction thermique (51) et une feuille de graphite (52) en matériau graphène, dans lequel
la feuille de graphite est fixée sur l'élément de conduction thermique, une extrémité de l'élément de conduction thermique est située dans le canal de refroidissement et l'autre extrémité de l'élément de conduction thermique est reliée à la portion de compresse froide.

10. Épilateur selon la revendication 9, dans lequel l'élément de conduction thermique comprend une chambre à vapeur (511) reliée à la portion de compresse froide et plusieurs dissipateurs thermiques (512) fixés sur une partie inférieure de la chambre à vapeur, dans lequel
la feuille de graphite est fixée sur une partie supérieure de la chambre à vapeur, la chambre à vapeur est fixée à une paroi interne du boîtier, une extrémité de la chambre à vapeur éloignée de la portion de compresse froide recouvre le second conduit de refroidissement, et les dissipateurs thermiques sont logés dans le second conduit de refroidissement et entrent en contact avec une partie de la chambre à vapeur couvrant le second conduit de refroidissement.

11. Épilateur selon la revendication 8, dans lequel le boîtier comprend un boîtier inférieur (24), un boîtier supérieur (25) recouvrant le boîtier inférieur, et un séparateur (26) configuré pour séparer le canal de refroidissement dans le premier conduit de refroidissement et le second conduit de refroidissement, dans lequel
le séparateur, le pilote de refroidissement, le tableau de commande, le composant épilatoire, la portion de compresse froide, la portion de conduction thermique et le composant de régulation de température sont tous serrés entre le boîtier inférieur et le boîtier supérieur.

12. Épilateur selon la revendication 11, dans lequel la coupelle réflectrice est montée sur le séparateur ;
dans lequel la coupelle réflectrice est pourvue d'une première rainure en forme U (331) dans une direction de longueur de la coupelle réflectrice, dans lequel
la direction de longueur de la coupelle réflectrice est identique à une direction d'écoulement du fluide de refroidissement, et dans lequel
le tube de lampe sans filament est situé dans la première rainure en forme U et s'appuie contre une paroi interne de la première rainure en forme U.

13. Épilateur selon la revendication 1, dans lequel le pilote de refroidissement est un turbo-ventilateur, le boîtier est en outre pourvu d'une entrée d'air (251), l'entrée d'air est disposée pour correspondre au pilote de refroidissement, le canal de refroidissement est situé à l'intérieur du boîtier, l'entrée d'air est en communication avec la sortie d'air par l'intermédiaire de l'orifice d'entraînement et du canal de refroidissement, et l'entrée d'air, la sortie d'air et la sortie de lumière sont toutes disposées sur un bord du boîtier.

14. Épilateur selon la revendication 1, dans lequel le pilote de refroidissement comporte en outre un orifice de guidage (13), dans lequel
l'orifice de guidage est en communication séparément avec l'entrée d'air et l'orifice d'entraînement ;
dans lequel l'épilateur comprend en outre un joint d'étanchéité élastique (80), dans lequel le joint d'étanchéité élastique comporte un trou traversant (81), et un espace est prévu entre le boîtier et une surface du pilote de refroidissement, et dans lequel
la surface susmentionnée fait face à l'entrée d'air ;
dans lequel le joint d'étanchéité élastique est serré entre le pilote de refroidissement et le boîtier pour bloquer l'espace, et l'entrée d'air, le trou traversant et l'orifice de guidage sont tous communiqués par séquence.

15. Épilateur selon la revendication 1, comprenant en outre un adaptateur d'alimentation (100), dans lequel
l'adaptateur d'alimentation est situé dans le boîtier et l'adaptateur d'alimentation est connecté électriquement au tableau de commande par l'intermédiaire d'un fil (110) ; et/ou
dans lequel l'épilateur comprend en outre un bouton de démarrage (200) configuré pour commander la mise sous tension de l'épilateur, dans lequel
un trou d'encastrement pénètre à travers le boîtier, et dans lequel
le bouton de démarrage est monté sur le trou d'encastrement et est connecté électriquement au tableau de commande.
